# EUROPEAN PATENT SPECIFICATION

(11) **EP 3 220 366 B1**
(45) Date of publication and mention of the grant of the patent: **25.05.2022**
(21) Application number: 17160898.7
(22) Date of filing: 14.03.2017
(51) Int. Cl.: G07F 17/00, G07F 11/54, G07F 13/00, A61L 2/10, A61L 2/07, A61L 2/18

(54) **DEVICE FOR DISPENSING FLUIDS SUCH AS WATER AND THE LIKE**
VORRICHTUNG ZUR AUSGABE VON FLÜSSIGKEITEN WIE ETWA WASSER UND DERGLEICHEN
DISPOSITIF POUR DISTRIBUER DES LIQUIDES TELS QUE L'EAU ET ANALOGUES

(30) Priority: 17.03.2016 IT UA20161747
(43) Date of publication of application: 20.09.2017
(73) Proprietor: DKR Drinkatering S.r.l., 20027 Rescaldina (MI) (IT)
(72) Inventor: DALLE FRATTE, Luigi, 20025 LEGNANO MI (IT)
(74) Representative: Modiano, Micaela Nadia

(56) References cited:
- WO-A2-2012/047916
- DE-A1-102012 207 665
- GB-A- 2 458 373
- US-A- 3 000 408
- US-A- 5 373 874
- US-A- 5 507 329
- US-A1- 2009 076 650
- US-A1- 2010 024 913
- US-B1- 7 571 586

## Description

The present invention relates to a device for dispensing fluids such as water and the like. More specifically, the invention relates to a device for dispensing fluids, for example water for bottles, milk and the like, which is done in outside environments.

As is known, nowadays devices are used for dispensing fluids such as water, milk and the like, which are located in outside environments and are accessible to users who wish to fill their containers with the above mentioned fluids.

The devices for dispensing fluids used currently use open dispensing compartments, especially in public distributors of water for bottles.

However, some solutions exist in which the dispensing compartments are closed by a door, usually pushed by a spring to move it to the closed position. This technical solution, although permitting an improvement from the point of view of hygiene in that the dispensing compartment is not exposed to external agents, is also the source of some nuisance for the user, in that there is an inconvenience generated by the spring of the door which forces the user to keep it open with one hand and to obtain the fluid with the other hand.

The inconvenience in question can be acceptable if the number of daily openings is limited, but it becomes unsustainable for example when water is drawn a very large number of times over the course of the day.

Furthermore, the size of the compartment determines the size of the door, with the corresponding dimensioning of the spring and the consequent inconvenience.

Furthermore, if the door is jammed in the open condition, the user may not be able to close it, or may simply forget to do so, thus defeating the point of the fact that the dispensing device has a door to protect the dispensing compartment.

Even furthermore, the dispensing compartment closed by a door has hermetic water seal problems, since the door is connected externally to the dispensing device, and therefore the gasket that provides the seal remains exposed to atmospheric agents, to tampering and to the innumerable actuations and it loses its efficacy over time, thus causing leaks when washing is carried out with the door closed so as to sanitize the internal environment.

In this case, washing with a door that is not perfectly hermetically sealed obviously causes the outflow of water.

Ultimately, the problem corresponding to the device for dispensing fluids in general is constituted by the fact that the dispensing compartment, and also the nozzles, must be periodically sanitized in order to be able to ensure the very highest standards of hygiene for the product that is dispensed.

For dispensing devices that do not close the dispensing compartment, the foregoing is utterly useless, since the compartment itself is subject to atmospheric agents and dirt in general and, in any case, to possible vandalism, and, being open, it does not make it possible to execute adequate automatic cleaning activities in total safety. In such case, the dispensing compartment does not have the adequate hygienic characteristics required.

But if there is a door to close dispensing compartment, the above mentioned drawbacks arise.

The aim of the present invention is to provide a device for dispensing fluids that makes it possible to protect the mode of dispensing from contact with atmospheric and potentially polluted agents.

US 5 373 874 discloses a device for dispensing fluids in which no movement means for the dispensing compartment are provided and wherein the compartment does not rotate about its own axis.

US 2009/076650 discloses an article storage and retrieval apparatus and vending machine.

US 2010/024913 discloses a refillable vending system and method.

US 5 507 329 discloses a distilled water dispensing apparatus.

GB 2458373 discloses a hygienic water dispensing apparatus

DE102012207665 discloses a vending machine for beverages with a rotating dispensing assembly.

Within this aim, an object of the present invention is to provide a device for dispensing fluids, which makes it possible to carry out a sanitization treatment of the dispensing compartment in total safety.

Another object of the present invention is to provide a device for dispensing fluids that prevents voluntary or involuntary contact by the users with the dispensing spout, and which also prevents exposure to vandalism and to dirt in general.

Another object of the present invention is to provide a dispensing device that is highly reliable, easily and practically implemented and low cost.

The invention is set out in the appended set of claims.

Further characteristics and advantages of the invention will become better apparent from the detailed description of a preferred, but not exclusive, embodiment of the dispensing device according to the present invention, which is illustrated by way of non-limiting example in the accompanying drawings, wherein:
Figure 1 is an exploded perspective view of the dispensing device according to the present invention;
Figure 2 is a perspective view of the dispensing device according to the present invention;

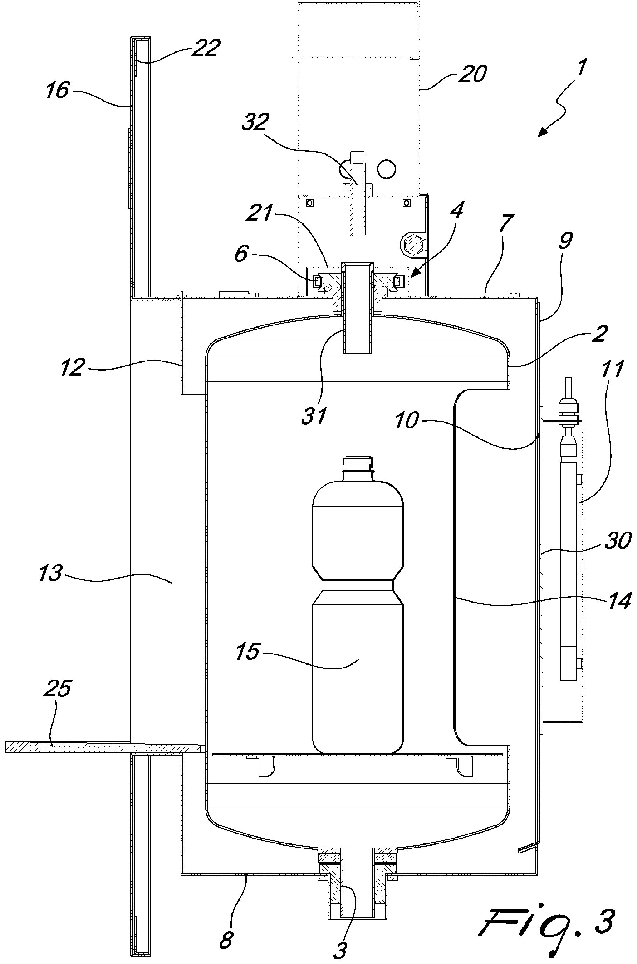

Figure 3 is a partially cross-sectional view of the dispensing device according to the present invention;

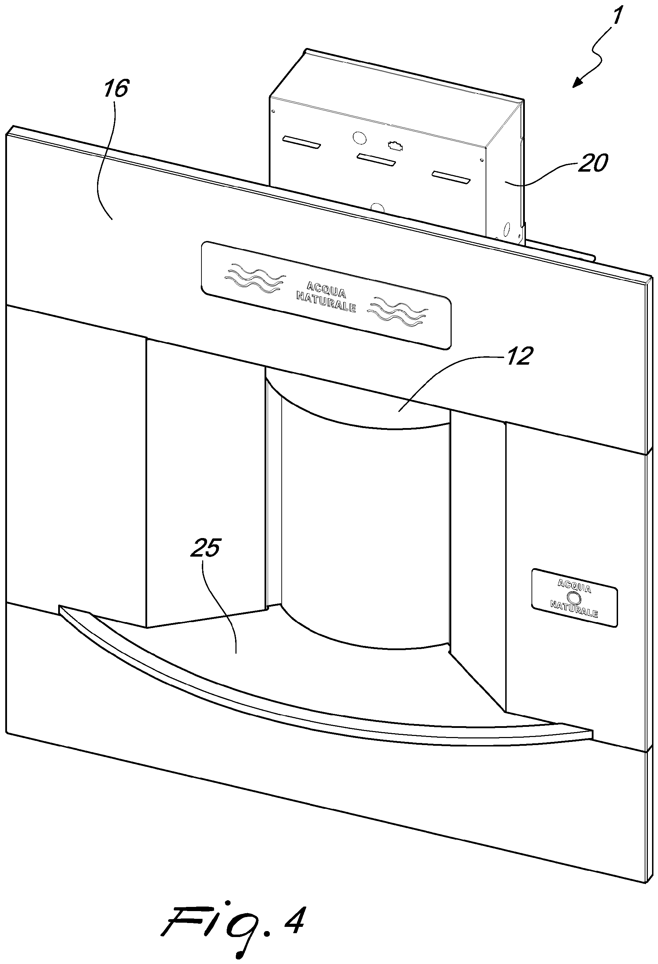

Figure 4 is a perspective view of the dispensing device according to the present invention in the closed condition;

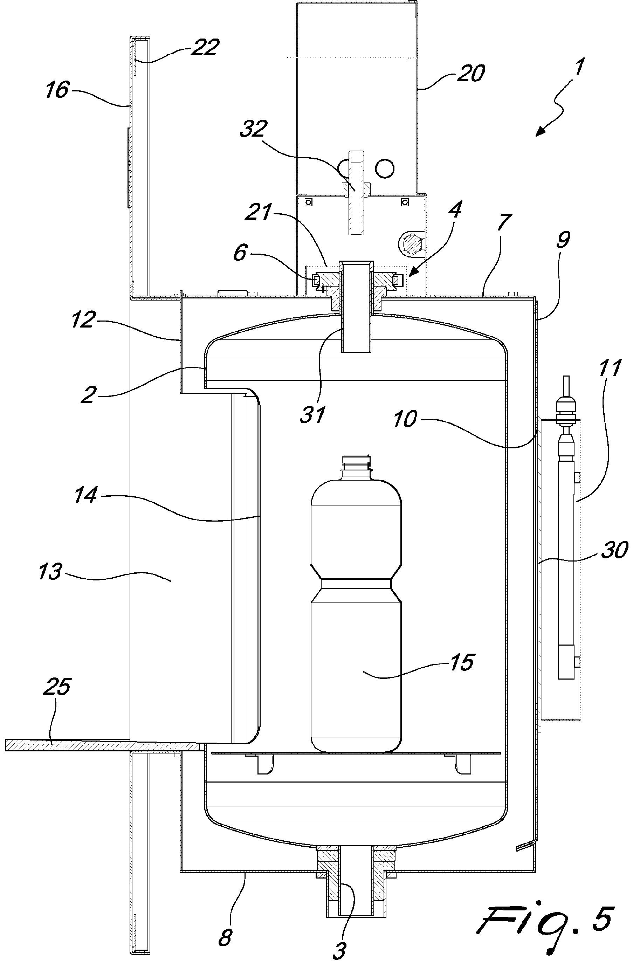

Figure 5 is a partially cross-sectional view of the dispensing device with the dispensing compartment in the open condition.

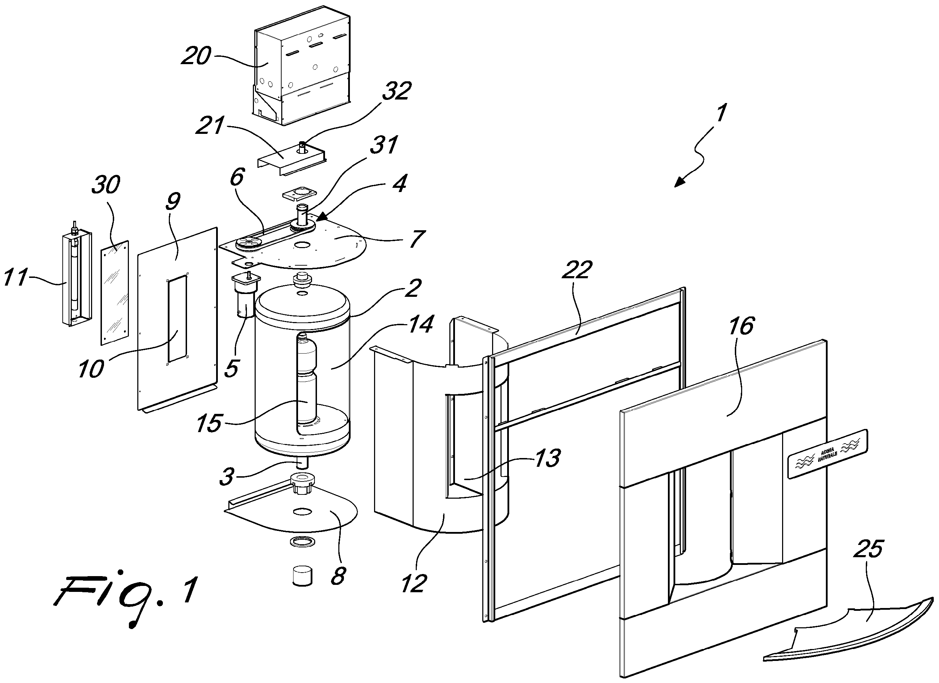

With reference to the figures, the dispensing device according to the present invention, generally designated by the reference numeral 1, comprises a container 2 provided with a dispensing compartment 14. The container 2 can rotate about a rotation axis that passes through, in Figure 1, a drain 3, and it is actuated by actuation means adapted to turn the container 2 and therefore the corresponding dispensing compartment 14.

The actuation means, generally designated by the reference numeral 4, can be pneumatic, hydraulic fluid-operated, or with an electric motor.

Figure 1 shows actuation means 4 provided with a drive engine 5 adapted to actuate a belt-and-pulley complex 6 in order to turn the container 2.

Conveniently, the dispensing compartment 14 is inserted between two covers, an upper cover 7 and a lower cover 8, and is delimited in a lower region by a partition 9 provided with an opening 10 through which at least one UV lamp 11 faces which is adapted to perform the sanitization of the dispensing compartment 2.

The UV lamp 11 is conveniently protected by a glass pane 30.

At the front, i.e. in a position arranged opposite from the partition 9, there is a covering element 12 provided with an opening 13 through which the dispensing compartment 14 of the container 2 faces.

The container 2 is adapted to accommodate a bottle 15 adapted to be filled with a liquid dispensed by a dispensing device which is directed into the container 2.

The device according to the invention is completed by a closing front panel 16 through which the covering element 12 faces.

The covering element 12 is advantageously shaped such that it accommodates the container 2, which can rotate inside the covering element 12.

Conveniently, the opening of the container 2, i.e. the dispensing compartment 14, affects a portion of the container 2, while the remaining portion is screened off. This makes it possible, when the container 2 is rotated, to pass from a condition of using the liquid dispensed by the dispensing device, i.e. when the dispensing compartment 14 is facing the front panel 16, to a position in which the container 2 is rotated so as to bring the closed portion of the container 2 to face the front panel 16.

In such condition, the dispensing compartment 14 instead faces toward the UV lamp 11 so as to be able to execute the sanitization treatment inside the dispensing compartment 14.

The reference numeral 20 designates a module board of the dispenser and the reference numeral 21 designates a covering housing of the belt-and-pulley complex 6.

The reference numeral 32 designates the dispensing nozzle, and the reference numeral 31 a spout which is passed through by the flow but without coming into contact with the product dispensed.

The dispensing nozzle 32 is arranged axially aligned with the drain 3, and the axis along which the dispensing nozzle 32 and the drain 3 are arranged may or may not coincide with the rotation axis of the container 2.

The reference numeral 22 designates instead a frame on which a front panel 16 of the dispensing device is fixed.

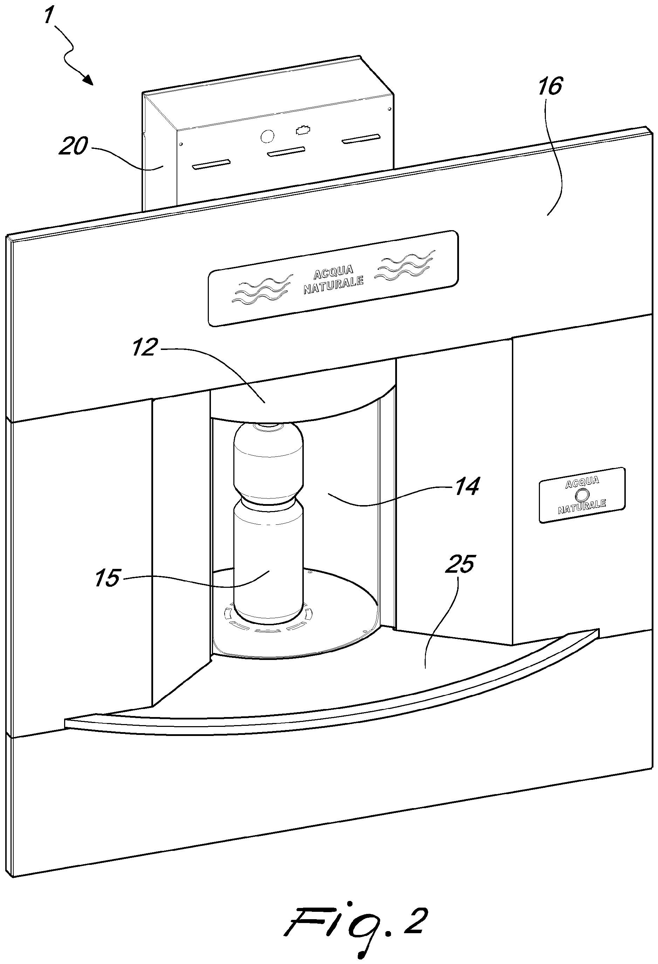

Finally, the reference numeral 25 designates a frontal resting surface which is better illustrated in Figure 2.

Operation of the dispensing device according to the invention is as follows.

By pressing a special button, or the dispensing button directly, or inserting a card if the device is thus controlled, the container 2 rotates, uncovering the internal part where the container or bottle 15 will be positioned.

The user, at this point, can proceed with the dispensing, with the dispensing compartment 14 exposed by the front panel 16 and therefore accessible to the user.

After having taken the liquid and concluded the dispensing cycle, after a period of activity, following a different event such as the pressing of a button and the removal of the card, the container 2 returns to the closed position, so that the dispensing compartment is not externally accessible.

In this position all the operations that are considered appropriate can be activated, including flushing, sanitization, washing, descaling, etc. in total safety because the user is protected from the closed part of the container 2.

Furthermore, the system remains protected from the rays of the UV lamp, from bad weather in general, from acts of vandalism, and from atmospheric conditions and/or from contact.

If the movement means of the container 2 are a rotating pneumatic actuator, such pneumatic actuator, conveniently powered, enables the rotation of the transmission pulley, which, by way of a belt, turns the container 2.

For a rotating hydraulic fluid actuator the principle is the same, and, for an electric gearmotor, the latter, conveniently controlled, enables the rotation of the transmission pulley which turns the container 2 by way of the belt.

Obviously other movement solutions are possible for turning the container 2.

The rotating pneumatic actuator and the rotating hydraulic fluid actuator make it possible, by appropriately adjusting the pressure of the power fluid, to control the speed and force of opening and closing.

Use of the electric gearmotor on the other hand makes it possible to control the speed of opening and closing of the container 2, similarly to the previous cases, and furthermore, by reading the absorption of current of the motor, it is possible to control the closing force and the crushing effect.

In order to increase safety, it is possible to use optical or pneumatic barriers in addition to the above mentioned movement means.

With the dispensing compartment 14 in the closed position, it is possible to set in motion different actions to keep the level of food and hygiene safety high, and these actions can be active or passive, automatic or manual:
- use of the UV lamp 11: the lamp can irradiate all the inside of the container;
- it is also possible to carry out different flushing actions in different ways;
- flushing of the dispensed product (to ensure the turnover of product in the event of inactivity, thus ensuring that the product that is dispensed is always fresh);
- flushing with a disinfectant fluid in order to sanitize the dispensing point only;
- flushing with a disinfectant fluid in order to sanitize the entire dispensing system;
- flushing with steam in order to sanitize the dispensing point;
- flushing with a descaling and/or detergent liquid in order to remove inorganic deposits and keep the dispensing compartment 14 clean;
- flushing with an algaecide liquid in order to keep the entire product drain area clean.

In practice it has been found that the dispensing device according to the present invention fully achieves the set aim and objects, since it makes it possible to dispense a desired liquid in total safety, with the dispensing compartment then being returned to the closed position at the end of the dispensing of the product.

The device, thus conceived, is susceptible of numerous modifications and variations, all of which are within the scope of the appended claims.

## Claims

1. A device (1) for dispensing fluids, such as water, milk and the like, comprising a container (2) provided with a dispensing compartment (14) and adapted to accommodate bottles (15) for containing a product; said container (2) being provided with a dispensing nozzle (32) for dispensing the product into said bottle; and a front panel (16) adapted to allow access to said dispensing compartment (14) by a user, **characterized in that** said container (2) is adapted to be turned by movement means (4),
wherein said container (2) is adapted to rotate about its own axis by virtue of said movement means,
wherein the dispensing point, defined by said dispensing nozzle (31), and the drain (3) coincide with the rotation axis of the container (2),
wherein pressing a special button, or a dispensing button, or inserting a card cause the container (2) to rotate to a condition in which said dispensing compartment (14) faces said front panel (16) and can be accessed from the outside of the device for a user to proceed with the dispensing; wherein after having concluded the dispensing cycle, after a period of activity, following a different event such as the pressing of a button and the removal of the card, the container 2 returns to a condition in which said dispensing compartment (14) rotates about its own axis so as to move said dispensing compartment (14) so that it is directed toward the inside of said dispensing device, thus preventing access by a user,
the device further **characterized in that** it has at least one UV lamp (11) adapted to irradiate said dispensing compartment (14) of said container (2) when said container (2) is rotated into the inactive condition, with said dispensing compartment (14) facing said lamp (11) and inaccessible to the user.

2. The device according to one or more of the preceding claims, **characterized in that** said movement means (4) comprise at least one among a pneumatic actuator, a hydraulic fluid actuator, and an electric motor, which are adapted to actuate a pulley and belt system or a lever system and/or a gear system.

3. The device according to one or more of the preceding claims, **characterized in that** said UV lamp (11) is arranged so as to face a slot (10) of a rear partition (9) of said dispensing device.

4. The device according to one or more of the preceding claims, **characterized in that** it comprises a covering element (12) adapted to accommodate said container (2), so as to cover said container, said container rotating within said covering element (12).

5. The device according to claim 4, **characterized in that** said covering element (12) has an opening (13) adapted to allow the facing arrangement of said dispensing compartment (14).

6. The device according to one or more of the preceding claims, **characterized in that** said container (2) is delimited in an upper region and in a lower region by two covers (7, 8).

## Patentansprüche

1. Eine Vorrichtung (1) zur Abgabe von Flüssigkeiten, wie etwa Wasser, Milch und dergleichen, die einen Behälter (2) umfasst, der mit einem Abgabebehälter (14) ausgestattet und ausgebildet ist, um Flaschen (15) zur Aufnahme eines Produkts aufzunehmen, wobei der Behälter (2) mit einer Abgabedüse (32), zur Abgabe des Produkts in die Flasche ausgestattet ist und eine Vorderplatte (16) ausgebildet um, einen Benutzer den Zugriff auf den Abgabebehälter (14) zu ermöglichen, **dadurch gekennzeichnet, dass** der Behälter (2) ausgebildet ist, um von Bewegungsmitteln (4) gedreht zu werden, wobei der Behälter (2) ausgebildet ist, um sich mit Hilfe der Bewegungsmittel um seine eigene Achse zu drehen, wobei der Abgabepunkt, der durch die Abgabedüse (31) bestimmt ist und der Ablass (3) mit der Drehachse des Behälters (2) zusammenfallen, wobei das Drücken einer speziellen Taste oder eines Abgabeknopfs, oder das Einführen einer Karte den Behälter (2) veranlassen, sich in einen Zustand zu drehen, indem der Abgabebehälter (14) der Vorderplatte (16) zugewandt ist und indem ein Benutzer, von außerhalb der Vorrichtung auf ihn Zugriff hat, um mit dem Abgabevorgang fortzufahren, wobei nach Abschluss des Abgabezyklus, nach einem Zeitraum der Aktivität und nach einem anderen Ereignis, wie zum Beispiel dem Drücken eines Knopfs und dem Entfernen der Karte, der Behälter (2) in einen Zustand zurückkehrt, in welchem der Abgabebehälter (14) sich um seine eigene Achse dreht, um den Abgabebehälter (14) zu bewegen, sodass er der Innenseite der Abgabevorrichtung zugewandt ist und dadurch den Zugriff durch einen Benutzer verhindert, wobei die Vorrichtung weiter **dadurch gekennzeichnet ist, dass** sie mindestens eine UV-Lampe (11) hat, ausgebildet um den Abgabebehälter (14) des Behälters (2) zu bestrahlen, wenn der Behälter (2) in den inaktiven Zustand gedreht ist, in welchem der Abgabebehälter (14) der Lampe (11) zugewandt und für den Benutzer unzugänglich ist.

2. Die Vorrichtung gemäß einem oder mehreren der obigen Ansprüche, **dadurch gekennzeichnet, dass** die Bewegungsmittel (4) mindestens eines von einem pneumatischen Aktor, einem Hydraulikflüssigkeitsaktor, und einem Elektromotor umfassen, die ausgebildet sind, um ein Riemenscheiben- und Riemen-System oder ein Hebelsystem und/oder ein Getriebe anzutreiben.

3. Die Vorrichtung gemäß einem oder mehreren der obigen Ansprüche, **dadurch gekennzeichnet, dass** die UV-Lampe (11) angeordnet ist, um einem Schlitz (10), einer hinteren Trennwand (9) der Abgabevorrichtung zugewandt zu sein.

4. Die Vorrichtung gemäß einem oder mehreren der obigen Ansprüche, **dadurch gekennzeichnet, dass** sie ein Abdeckelement (12) umfasst, das ausgebildet ist, um den Behälter (2) aufzunehmen und um ihn abzudecken, wobei sich der Behälter innerhalb des Abdeckelements (12) dreht.

5. Die Vorrichtung gemäß Anspruch 4, **dadurch gekennzeichnet, dass** das Abdeckelement (12) eine Öffnung (13) hat, die ausgebildet ist, um die zugewandte Anordnung des Abgabebehälters (14) zu ermöglichen.

6. Die Vorrichtung gemäß einem oder mehreren der obigen Ansprüche, **dadurch gekennzeichnet, dass** der Behälter (2) in einem oberen und einem unteren Bereich durch zwei Deckplatten (7, 8) begrenzt ist.

## Revendications

1. Dispositif (1) pour distribuer des liquides, comme de l'eau, du lait et autres, comprenant un récipient (2) pourvu d'un compartiment de distribution (14) et adapté pour recevoir des bouteilles (15) destinées à contenir un produit ; ledit récipient (2) étant pourvu d'une buse de distribution (32) pour distribuer le produit dans ladite bouteille ; et un panneau avant (16) adapté pour permettre l'accès audit compartiment de distribution (14) par un utilisateur, **caractérisé en ce que** ledit récipient (2) est adapté pour être mis en rotation par un moyen de mouvement (4),
dans lequel ledit récipient (2) est adapté pour tourner autour de son propre axe grâce audit moyen de mouvement,
dans lequel le point de distribution, défini par ladite buse de distribution (31), et le drain (3) coïncident avec l'axe de rotation du récipient (2),
dans lequel une pression exercée sur un bouton spécial, ou un bouton de distribution, ou l'insertion d'une carte provoquent la rotation du récipient (2) jusqu'à un état dans lequel ledit compartiment de distribution (14) se trouve en face dudit panneau avant (16) et peut être atteint depuis l'extérieur du dispositif pour qu'un utilisateur réalise la distribution ;
dans lequel, après avoir fini le cycle de distribution, après une période d'activité, faisant suite à un événement différent tel que l'actionnement d'un bouton et le retrait de la carte, le récipient (2) retourne à un état dans lequel ledit compartiment de distribution (14) tourne autour de son propre axe afin de déplacer ledit compartiment de distribution (14) pour l'orienter vers l'intérieur dudit dispositif de distribution, empêchant ainsi l'accès par un utilisateur,
le dispositif étant **caractérisé en outre en ce qu'**il comporte au moins une lampe ultraviolette (11) adaptée pour irradier ledit compartiment de distribution (14) dudit récipient (2) lorsque ledit récipient (2) tourne jusqu'à l'état inactif, ledit compartiment de distribution (14) se trouvant face à ladite lampe (11) et étant inaccessible pour l'utilisateur.

2. Dispositif selon l'une ou plusieurs des revendications précédentes, **caractérisé en ce que** ledit moyen de mouvement (4) comprend au moins un élément parmi un actionneur pneumatique, un actionneur hydraulique et un moteur électrique, qui sont adaptés pour actionner un système à poulie et courroie ou un système de levier et/ou un système d'engrenages.

3. Dispositif selon l'une ou plusieurs des revendications précédentes, **caractérisé en ce que** ladite lampe ultraviolette (11) est disposée de façon à se trouver en face d'une fente (10) d'une cloison arrière (9) dudit dispositif de distribution.

4. Dispositif selon l'une ou plusieurs des revendications précédentes, **caractérisé en ce qu'**il comprend un élément de protection (12) adapté pour recevoir ledit récipient (2), de manière à couvrir ledit récipient, ledit récipient tournant sans dépasser dudit élément de protection (12).

5. Dispositif selon la revendication 4, **caractérisé en ce que** ledit élément de protection (12) comporte une ouverture (13) adaptée pour permettre la mise en place en regard dudit compartiment de distribution (14).

6. Dispositif selon l'une ou plusieurs des revendications précédentes, **caractérisé en ce que** ledit récipient (2) est délimité dans une région supérieure et dans une région inférieure par deux couvercles (7, 8).
